# NEUE EUROPÄISCHE PATENTSCHRIFT

(11) **EP 0 552 227 B2**
(45) Veröffentlichungstag und Bekanntmachung des Hinweises auf die Entscheidung über den Einspruch: **04.11.1998**
(45) Hinweis auf die Patenterteilung: 11.01.1995
(21) Anmeldenummer: 91917940.8
(22) Anmeldetag: 07.10.1991
(51) Int. Cl.: A61K 9/18, A61K 9/16, A61K 9/20, A61K 31/34

(54) **LÖSUNGSMITTELFREIE, ORAL ZU VERABREICHENDE PHARMAZEUTISCHE ZUBEREITUNG MIT VERZÖGERTER WIRKSTOFFFREISETZUNG UND VERFAHREN ZU DEREN HERSTELLUNG**
ORALLY ADMINISTERED SOLVENT-FREE PHARMACEUTICAL PREPARATION WITH DELAYED ACTIVE-SUBSTANCE RELEASE, AND A METHOD OF PREPARING THE PREPARATION
PREPARATION PHARMACEUTIQUE EXEMPTE DE SOLVANTS, ADMINISTREE ORALEMENT, A LIBERATION RETARDEE DU PRINCIPE ACTIF, AINSI QUE PROCEDE POUR SA FABRICATION

(30) Priorität: 08.10.1990 DE 4031881
(43) Veröffentlichungstag der Anmeldung: 28.07.1993
(73) Patentinhaber: SCHWARZ PHARMA AG, D-40789 Monheim/Rhld. (DE)
(72) Erfinder: MÜNCH, Ulrich, D-4019 Monheim (DE); MIKA, Hans-Jürgen, D-5300 Bonn 3 (DE); EMSCHERMANN, Bernhard, D-4000 Düsseldorf 13 (DE); SCHMIDT, Rainer, D-5090 Leverkusen 3 (DE); SCZEPANIK, Bernhard, D-5653 Leichlingen (DE)
(74) Vertreter: von Füner, Alexander, Prof.h.c. Dr.
(86) Internationale Anmeldenummer: DE9100791
(87) Internationale Veröffentlichungsnummer: WO9205774

(56) Entgegenhaltungen:
- EP-A- 240 904
- EP-A- 240 906
- EP-A- 358 105
- EP-A- 0 263 083
- EP-A- 0 337 256
- DE-A- 1 229 248
- FR-A- 2 140 131
- US-A- 3 308 217
- Pharmazeutische Industrie, Bd. 33, Nr. 12, 1971, Aulendorf, Seiten 903-913; Ritschel W.A. et al.: "Verfahrenslehre und mechanische Verfahrenstechnik für die Fabrikation von Arzneimitteln"
- Chemical Abstracts, vol. 101, no. 1, 2. Juli 1984, Colombus, Ohio, US; abstract no. 60197H, Waechter, W. et al.: "Rational use of DSC in pharmaceutical quality control as illustrated by Isosorbide dinitrate", Seite 314, Spalte 1

## Beschreibung

Die Erfindung betrifft eine lösungsmittelfreie, oral zu verabreichende pharmazeutische Zubereitung mit verzögerter Wirkstofffreisetzung und Verfahren zu deren Herstellung, bei denen auf die Verwendung von Lösungsmitteln verzichtet wird.

In der pharmazeutischen Technologie sind eine Vielzahl von Möglichkeiten zur Herstellung von oralen Retardarzneiformen bekannt. Unterschiedliche galenische Prinzipien finden dabei Anwendung. Der Wirkstoff kann modifiziert werden oder Diffusionsbarrieren können errichtet werden. Insbesondere das letztgenannte Prinzip findet in der gängigen Praxis häufige Anwendung. So wird der Wirkstoff mit Polymersystemen umhüllt oder der Arzneistoff wird in Matrixsysteme eingebunden, aus denen er dann verzögert freigesetzt wird. Hierbei wird vornehmlich mit organischen Lösungsmitteln gearbeitet.

Als gängige Lösungsmittel werden beispielsweise chlorierte Kohlenwasserstoffe, insbesondere Methylenchlorid, Aceton oder Alkohol eingesetzt.

Sie werden benötigt, um den jeweils eingesetzten retardierenden matrixbildenden Hilfsstoff zu lösen und den Wirkstoff sowie die übrigen Hilfsstoffe damit zu überziehen oder zu verkleben. Weiterhin dienen sie als Feuchtmittel zum Granulieren.

Während bisher bei der Entwicklung von Retardpräparaten das Erreichen verschiedener Zielsetzungen im Vordergrund stand, nämlich u.a.
- Aufrechterhaltung therapeutischer Plasmakonzentrationen unter Vermeidung von Wirkungsschwankungen über längere Zeit;
- Vermeidung von zu hohen Plasmakonzentrationsspitzen um unerwünschte Wirkungen zu reduzieren:
- Verlängerung des Dosierungsintervalles zur Erreichung einer verbesserten Patienten-Compliance, richtet sich nunmehr das Interesse darauf, Retardpräparate ohne Verwendung von Lösungsmitteln unter Erreichen der oben genannten Zielsetzungen herstellen zu können.

Auf Grund veränderten Umweltbewußtseins besteht jetzt ein Bedarf, Retardarzneiformen ohne Verwendung von Lösungsmitteln herstellen zu können. Behördliche Auflagen zur Entsorgung der verwendeten Lösungsmittel und insbesondere die Vermeidung toxikologischen Risikos von in den Arzneiformulierungen enthaltenen Lösemittelrestmengen. z.B. chlorierte Kohlenwasserstoffe, zwingen zum Verzicht der Verwendung von Lösungsmitteln bei der Herstellung von Retardarzneiformen.

Weiterhin hat sich inzwischen ergeben, daß innerhalb der Kategorie "orale Retardarzneiformen" multiple Arzneiformen (Multiple Unit Dosage Forms) im Vergleich zu monolithischen Arzneiformen (Singe Units) Vorteile aufweisen. Insbesondere aus biopharmazeutischer Sicht sind Multiple Unit Dosage Forms Single Units vorzuziehen. Multiple Unit-Präparate weisen beispielsweise kürzere Magenpassagezeiten auf und ermöglichen eine rasche und gleichförmige Verteilung der definierten Untereinheiten über den gesamten Gastrointestinaltrakt. Dadurch können lokale Irritationen durch hohe Arzneistoffkonzentrationen vermieden werden. Gleichzeitig wird die Gefahr eines "dose dumping" verringert.

Die Schwankungen der AUC-Werte und die Streuungen der relevanten Zielgrößen lag time, Cₘₐₓ und tₘₐₓ sind geringer.

Allerdings ist die Herstellung multipler Retardarzneiformen nach den bisherigen Herstellmethoden unter Verwendung von Lösungsmitteln noch immer mit Risiken der ordnungsgemäßen pharmazeutischen Qualität der Zubereitungen behaftet. Die Reproduzierbarkeit guter pharmazeutischer Qualität innerhalb eine Charge (Chargenhomogenität) sowie von Charge zu Charge (Chargenkonformität) ist nicht immer gewährleistet. (H. Blume, Biopharmaz. Aspekte von Multiple Unit Dosage Forms, 1988) Obwohl bereits eine ganze Reihe von Arzneiformen für die Langzeitabgabe von Wirkstoffen bekannt ist, besteht noch weiterhin ein Bedürfnis nach verbesserten Arzneiformen.

Aufgabe der vorliegenden Erfindung ist es, eine oral zu verabreichende pharmazeutische Zubereitung mit verzögerter Wirkstofffreisetzung ohne darin enthaltene Lösungsmittelrestmengen zur Verfügung zu stellen, die sowohl zu multiplen Arzneiformen (Multiple Unit Dosage Forms) als auch zu monolithischen Arzneiformen (Single Units) weiterverarbeitet werden kann. Weiterhin ist es Aufgabe der Erfindung, Verfahren zur Herstellung einer derartigen pharmazeutischen Zubereitung mit verzögerter Wirkstofffreisetzung zur Verfügung zu stellen, die die Verwendung von Lösungsmitteln ausschließen.

Gleichzeitig sollen unter Aufrechterhaltung guter pharmazeutischer Qualität die Menge des eingesetzten Wirkstoffes in weiten Grenzen variiert werden können und die Freisetzung des Wirkstoffes gezielt steuerbar sein.

Gegenstand der Erfindung ist eine oral zu verabreichende pharmazeutische Zubereitung mit verzögerter Wirkstofffreisetzung wobei die Zubereitung aus einem lösungsmittelfreien, erkalteten Schmelzgranulat besteht, das Isosorbiddinitrat oder Isosorbid-5-mononitrat, sowie Polyvinylacetat und hochdisperses Siliciumdioxid enthält.

Der geschmolzene Wirkstoff dient als Lösungsmittel für den zu lösenden, die Retardierung bewirkenden Hilfsstoff. Insoweit ist die Wirkstoffschmelze das Lösungsmittel für den Hilfsstoff. Beide zusammen ermöglichen die Feuchtgranulation zur Ausbildung einer Retardmatrix.

Als Arzneiformen kommen feste Darreichungsformen wie Tabletten, Pellets, Kapseln, Granula und Dragees in Betracht. In hervorragender Weise sind Pellets und zu schnell zerfallenden Tabletten gepreßte Granula geeignet. Diese Formen unterliegen einer reproduzierbaren Bewegungskinetik im Magen-Darm-Kanal. Die Transitzeit vom Verlassen des Magens bis zum Erreichen des Colons ist gut vorhersehbar und unabhängig von der Nahrungsaufnahme (S.S. Davis et al., Int. J. Pharmaceutics, 21, 331 - 340 (1984)). Sie beträgt typisch 3 - 4 Std. (J.C. Hardy. J. Nucl. Med., 25, 59 (1984)).

Arzneiformen der erfindungsgemäßen Zubereitung zeichnen sich dadurch aus, daß sie in einfacher, dem Fachmann bekannter Weise den jeweiligen Erfordernissen der Wirkstofffreisetzung angepaßt werden können

Die Arzneiform ist eine Tablette, ein Pellet, eine Kapsel oder ein Dragee.

Die erfindungsgemäße pharmazeutische Zubereitung kann hergestellt werden, indem eine Mischung, bestehend aus dem Wirkstoff und dem Hilfsstoff geschmolzen wird, bis zum Entstehen einer homogenen, gleichmäßig durchfeuchteten Masse geknetet und anschließend granuliert wird.

Die erfindungsgemäße pharmazeutische Zubereitung kann auch durch das Verfahren der Schmelzextrusion hergestellt werden.

Im folgenden wird die Erfindung näher beschrieben.

Durch Veränderung der Mengenverhältnisse von eingesetztem Wirkstoff oder Wirkstoffgemisch und Hilfsstoff bzw. Hilfsstoffen oder Variation des Verhältnisses von Masse zur Oberfläche der Formlinge können die Eigenschaften der pharmazeutischen Zubereitung bestimmt werden.

Die Verwendung von hochdispersem Siliciumdioxid in Verbindung mit dem wasserunlöslichen Matrixbilder, beispielsweise Polyvinylacetat (PVA) führt zu einem stabilen Matrixskelett.

Das Skelett sorgt für Strukturstabilität und hält die Poren des Schwammes offen. Die Klebrigkeit ist herabgesetzt.

Zuschläge wasserlöslicher Stoffe wie Lactose erhöhen die Porosität und somit die Freisetzungsgeschwindigkeit des Wirkstoffes. Talkum als mechanisches Hindernis verlängert die Diffusionswege und führt zu einer Verlangsamung der Wirkstofffreisetzung.

In der Wärme ist die aus geschmolzenem Wirkstoff und darin gelöstem Hilfsstoff bestehende Masse knet- und verformbar, so daß eine völlig homogene Mischung bzw. "Befeuchtung" erreicht werden kann. Bei tiefer Temperatur, insbesondere bei Körpertemperatur ist dennoch völlige Stabilität der Struktur gegeben. Diese Stabilität bleibt auch in Gegenwart von Wasser bzw. simulierten Verdauungssäften erhalten.

Aufgrund des bei hoher Temperatur vorhandenen thermoplastischen Verhaltens kann zur Herstellung der Mischung das Verfahren der Schmelzextrusion genutzt werden.

### Ausführungsbeispiel 1

2.5 g einer ISDN/Lactose-Mischung, bestehend aus 40 % ISDN und 60 % Lactose wurden mit 0,7 g abgesiebtem Feinanteil Polyvinylacetat (Handelstyp Vinapas B 5 spezial) und 0.6 g hochdispersem Siliciumdioxid (Aerosil 200®) trocken gemischt, durch ein Teesieb gesiebt, in einer Reibschale in einem Trockenschrank von ca. 80°C erwärmt und mit dem Pistill geknetet, bis eine homogene, gleichmäßig durchfeuchtete zähe Masse entstanden war. Während des Abkühlens wurde eine Spur ISDN/Lactose-Mischung 40/60 zur Impfung darüber gestreut und mit dem Pistill geknetet. Die Kristallisation beim Kneteh führte in kurzer Zeit zu einem recht festen Material, das allmählich weiter aushärtete. Es verhielt sich etwas Plastisch, brach aber auch leicht. Kleine Stücke wurden auf einer beheizten Metallplatte bei 40 bis 50°C zu dünnen Strängen ausgerollt und nach dem Erkalten in kleine Stücke geschnitten. Am nächsten Tag wurden die Stücke durch ein 1-mm-Sieb gegeben. Der Feinanteil wurde mit dem Teesieb abgesiebt.

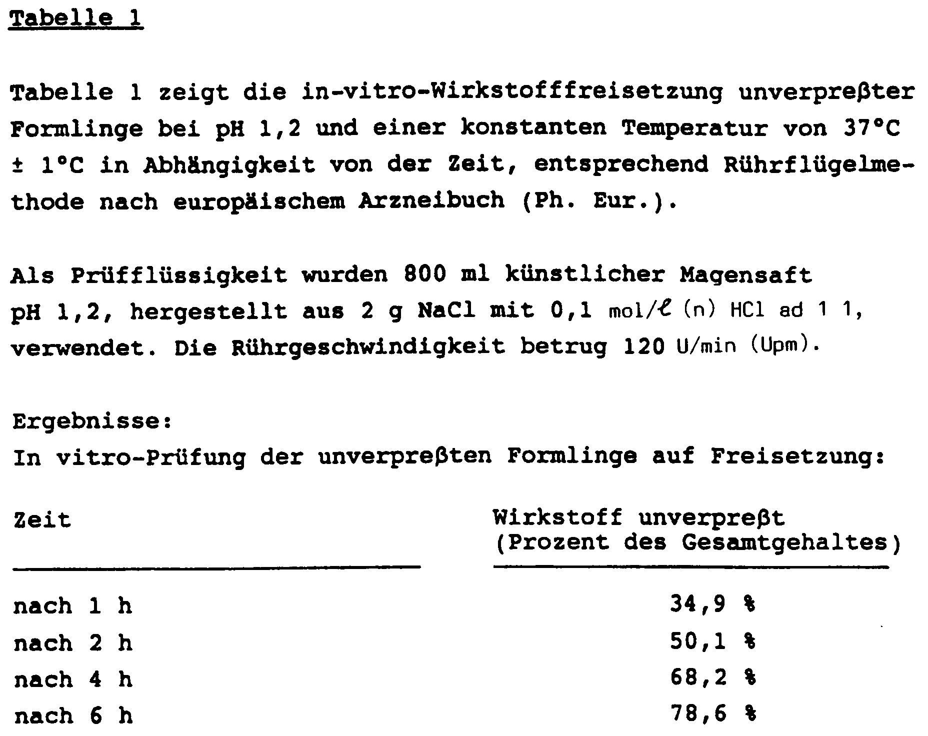

Diese Freisetzungsergebnisse zeigen die Eignung der Formlinge zur Verarbeitung zu Multiple Unit Dosage Forms, z.B. Kapseln.

### Ausführungsbeispiel 2

Herstellung von Pellets und Verarbeitung zu schnell zerfallenden Tabletten.

2,5 g einer ISDN/Lactose-Mischung, bestehend aus 40 % ISDN und 60 % Lactose wurden mit 0,7 g abgesiebtem Feinanteil Polyvinylacetat (Handelstyp Vinapas B 5 spezial) und 0,6 g hochdispersem Siliciumdioxid (Aerosil 200®) trocken gemischt, durch ein Teesieb gesiebt in einer Reibschale in einem Trockenschrank von ca. 80°C erwärmt und mit dem Pistill geknetet, bis eine homogene, gleichmäßig durchfeuchtete, zähe Masse entstanden war. Während des Abkühlens wurde eine Spur ISDN/Lactose-Mischung 40/60 zur Impfung darüber gestreut und mit dem Pistill geknetet. Die Kristallisation beim Kneten führte in kurzer Zeit zu einem recht festen Material, das allmählich weiter aushärtete. Es verhielt sich etwas plastisch, brach aber auch leicht. Kleine Stücke wurden auf einer beheizten Metallplatte bei 40 bis 50°C zu dünnen Strängen ausgerollt, nach dem Erkalten in kleine Stücke geschnitten und durch ein 1 mm Sieb gegeben. Den Pellets wurden weitere zur Tablettierung übliche Zuschläge zugemischt. Anschließend wurde mit einem Preßdruck von 2 t tablettiert.

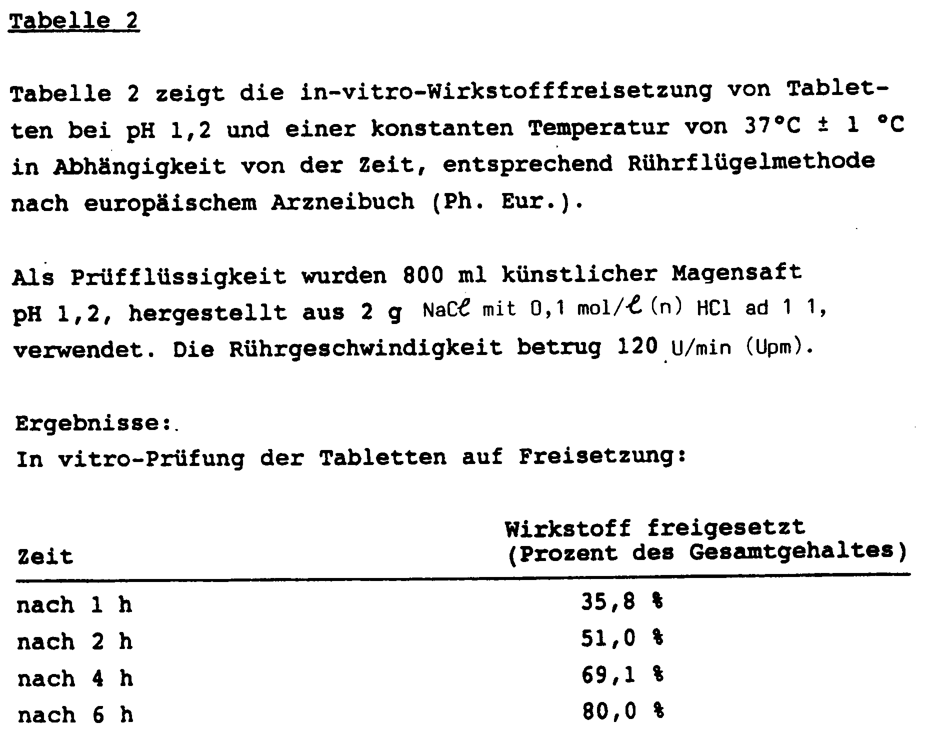

### Ausführungsbeispiel 3

Herstellung der lösungsmittelfreien pharmazeutischen Zubereitung nach dem Schmelzextrusionsverfahren und Verarbeitung zu Matrixtabletten.

2,5 kg ISDN/Lactose-Mischung, in der Zusammensetzung 40 % ISDN und 60 % Lactose, wurden mit abgesiebtem Feinanteil 2,75 kg Talkum, 0,11 kg Magnesiumstearat, 6,35 kg Lactose, 0,75 kg Polyvinylacetat und 0,025 kg Eisenoxid gemischt und nochmals durch ein Sieb 3 mm gesiebt. Die Mischung wurde anschließend in einen Doppelschneckenschmelzextruder verbracht, in dem sie unter Wärmeeinwirkung in Zonen steigender Temperatur von 60°C bis 100°C zu einer homogenen, gleichmäßig durchfeuchteten zähen Masse geknetet wurde. Der ausgeworfene, abgekühlte Strang wurde zu dünnen Schnitzeln verarbeitet. Diese wurden durch einen Frewitt-Sieb 1,25 mm gesiebt. Das erhaltene Granulat wurde unter einem Preßdruck von 2 t zu Tabletten gepreßt.

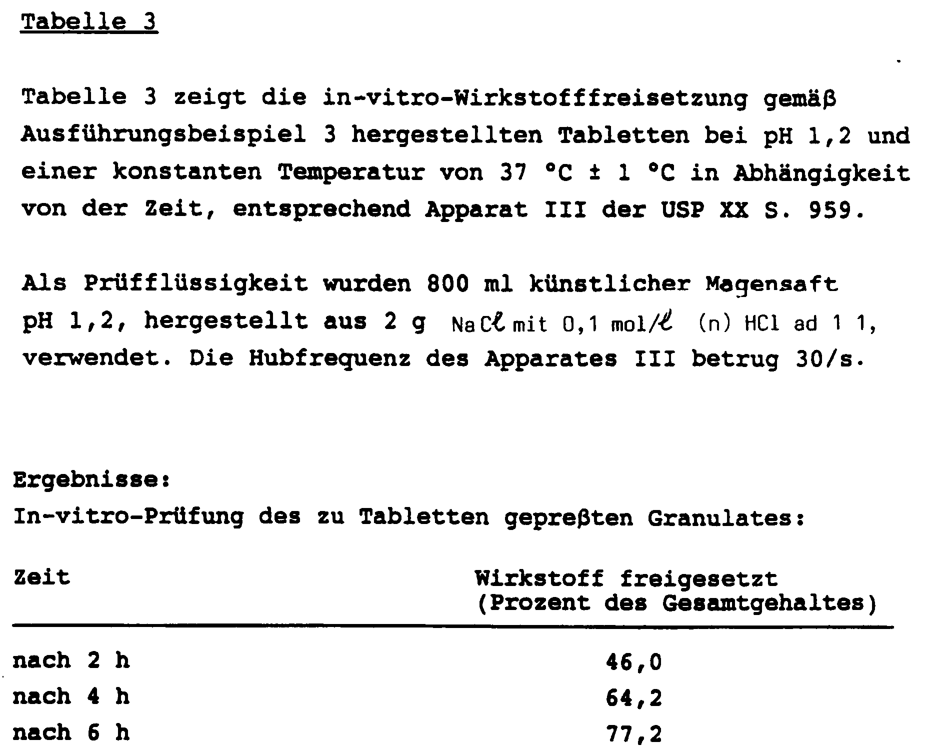

## Patentansprüche

1. Oral zu verabreichende pharmazeutische Zubereitung mit verzögerter Wirkstofffreisetzung, **dadurch gekennzeichnet**, daß die Zubereitung aus einem lösungsmittelfreien, erkalteten Schmelzgranulat besteht, das Isosorbiddinitrat oder Isosorbid-5-mononitrat, sowie Polyvinylacetat und hochdisperses Siliciumdioxid enthält.

2. Zubereitung nach Anspruch 1, **dadurch gekennzeichnet**, daß sie zusätzlich Talkum enthält.

3. Zubereitung nach Anspruch 1 oder 2, **dadurch gekennzeichnet**, daß sie zusätzlich Lactose enthält.

4. Verfahren zur Herstellung der Zubereitung nach Anspruch 1, **dadurch gekennzeichnet**, daß Isosorbiddinitrat oder Isosorbid-5-mononitrat mit Polyvinylacetat und hochdispersem Siliciumdioxid vermischt, die so erhaltene Mischung geschmolzen, bis zum Entstehen einer homogenen, gleichmäßig durchfeuchteten Masse geknetet und anschließend granuliert wird.

5. Verfahren nach Anspruch 4, **dadurch gekennzeichnet**, daß der Mischung zusätzlich Talkum zugegeben wird.

6. Verfahren nach Anspruch 4 oder 5, **dadurch gekennzeichnet**, daß der Mischung zusätzlich Lactose zugegeben wird.

7. Verfahren zur Herstellung der Zubereitung nach Anspruch 1, **dadurch gekennzeichnet**, daß Isosorbiddinitrat oder Isosorbid-5-mononitrat mit Polyvinylacetat und hochdispersem Siliciumdioxid vermischt, die erhaltene Mischung der Schmelzextrusion unterworfen und anschließend granuliert wird.

8. Verfahren nach Anspruch 7, **dadurch gekennzeichnet**, daß der Mischung zusätzlich Talkum zugegeben wird.

9. Verfahren nach Anspruch 7 oder 8, **dadurch gekennzeichnet**, daß der Mischung zusätzlich Lactose zugegeben wird.

10. Verfahren nach einem der Ansprüche 7 bis 9, **dadurch gekennzeichnet**, daß die Mischung in einem Temperaturbereich von 60°C bis 110°C schmelzextrudiert wird.

## Claims

1. Pharmaceutical preparation to be administered orally, having delayed release of active compound; characterized in that the preparation consists of a solvent-free, cooled melt granulate which contains isosorbide dinitrate or isosorbide 5-mononitrate, and also polyvinyl acetate and highly disperse silica.

2. Preparation according to Claim 1, characterized in that it additionally contains talc.

3. Preparation according to Claim 1 or 2, characterized in that it additionally contains lactose.

4. Process for the production of the preparation according to Claim 1, characterized in that isosorbide dinitrate or isosorbide 5-mononitrate is mixed with polyvinyl acetate and highly disperse silica, the mixture thus obtained is fused, kneaded until a homogeneous, uniformly moistened mass is formed and then granulated.

5. Process according to Claim 4, characterized in that talc is additionally added to the mixture.

6. Process according to Claim 4 or 5, characterized in that lactose is additionally added to the mixture.

7. Process for the production of the preparation according to Claim 1, characterized in that isosorbide dinitrate or isosorbide 5-mononitrate is mixed with polyvinyl acetate and highly disperse silica, and the mixture obtained is subjected to melt extrusion and then granulated.

8. Process according to Claim 7, characterized in that talc is additionally added to the mixture.

9. Process according to Claim 7 or 8, characterized in that lactose is additionally added to the mixture.

10. Process according to one of Claims 7 to 9, characterized in that the mixture is melt-extruded in a temperature range from 60°C to 110°C.

## Revendications

1. Préparation pharmaceutique administrée oralement et à libération retardée du principe actif, ***caractérisée en ce que*** la préparation est constituée d'un granulé de fusion refroidi, exempt de solvants, qui contient du dinitrate d'isosorbide ou du 5-mononitrate d'isosorbide, ainsi que de l'acétate de polyvinyle et du dioxyde de silicium à haute dispersion.

2. Préparation selon la Revendication 1, ***caractérisée en ce qu***'elle contient en plus du talc.

3. Préparation selon la Revendication 1 ou 2, ***caractérisée en ce qu***'elle contient en plus du lactose.

4. Procédé de fabrication d'une préparation selon la Revendication 1, ***caractérisé en ce que*** du dinitrate d'isosorbide ou du 5-mononitrate d'isosorbide est mélangé à de l'acétate de polyvinyle et à du dioxyde de silicium à haute dispersion, le mélange ainsi obtenu est fondu, malaxé jusqu'à obtention d'une masse homogène uniformément humidifiée, puis granulé.

5. Procédé selon la Revendication 4, ***caractérisé en ce que*** le mélange contient en plus du talc.

6. Procédé selon la Revendication 4 ou 5, ***caractérisé en ce que*** le mélange contient en plus du lactose.

7. Procédé de fabrication d'une préparation selon la Revendication 1, ***caractérisé en ce que*** du dinitrate d'isosorbide ou du 5-mononitrate d'isosorbide est mélangé à de l'acétate de polyvinyle et à du dioxyde de silicium à haute dispersion, le mélange ainsi obtenu est soumis à une extrusion de matière fondue, puis est granulé.

8. Procédé selon la Revendication 7, ***caractérisé en ce que*** le mélange contient en plus du talc.

9. Procédé selon la Revendication 7 ou 8, ***caractérisé en ce que*** le mélange contient en plus du lactose.

10. Procédé selon l'une quelconque des Revendications 7 à 9, ***caractérisé en ce que*** le mélange est extrudé à l'état fondu dans une plage de température de 60°C à 110°C.
